# EUROPEAN PATENT APPLICATION

(11) **EP 2 959 869 A1**
(43) Date of publication of application: **30.12.2015**
(21) Application number: 15172995.1
(22) Date of filing: 19.06.2015
(51) Int. Cl.: A61F 5/01

(54) **ORTHOPEDIC BRACE APPLYING VARIABLE TENSION DURING JOINT RANGE OF MOTION ACTIVITY**

(30) Priority: 28.06.2014 US 201462018575 P; 12.06.2015 US 201514738779; 12.06.2015 US 201514738774
(71) Applicant: Breg, Inc., Carlsbad, CA 92010 (US)
(72) Inventor: Fedon, Shane C., California 92024 (US)
(74) Representative: D Young & Co LLP

(57) **Abstract**

An orthopedic brace for a body joint has a first longitudinal support, a second longitudinal support and a hinge joint rotatably connecting the longitudinal supports, thereby rendering them positionable at different hinge joint angles. The brace also has a tensioner attached at its opposite ends to the first and second longitudinal supports which intermediately engages a cam positioned at the hinge joint. The tensioner applies a variable elastic tensioning force to the longitudinal supports which varies as a function of the hinge joint angle and biases the longitudinal supports toward a position corresponding to a desired hinge joint angle.

## Description

This a non-provisional patent application claiming the priority of Provisional Patent Application Serial No. 62/018,575, filed on June 28, 2014, which is incorporated herein by reference.

### BACKGROUND

The present disclosure relates generally to orthopedic braces, and in a more particular embodiment but not exclusively, to orthopedic braces for supplementing a human body joint.

Orthopedic braces embody a broad range of apparatuses, each having the common purpose of supporting and/or stabilizing a skeletal body joint on the body of a wearer. The orthopedic brace may serve either a preventative role or a remedial role. In a preventative role, the brace provides added support and stability to a healthy body joint, thereby reducing the risk of injury when the joint is subjected to undue stress. In a remedial role, the brace supports and stabilizes a body joint which has been weakened by injury or other infirmity, thereby reinforcing the body joint and reducing the risk of further injury while the body joint is rehabilitated. One conventional type of orthopedic brace is a hip brace which is specifically configured for supporting and stabilizing the hip joint.

An example conventional hip brace is disclosed in U.S. Patent No. 4,481,941 to Rolfes. The present disclosure recognizes the need for an orthopedic brace for a human body joint, such as a hip joint, which represents an improvement over conventional orthopedic braces and enhances the performance of the body joint.

### SUMMARY

Particular aspects and embodiments are set out in the appended independent and dependent claims.

Viewed from some perspectives, the present teachings can provide an orthopedic brace for a human body joint which satisfies a number of wearer treatment functions including a rehabilitative, therapeutic and/or preventative treatment function. Accordingly, some implementations of the present teachings can provide an orthopedic brace exhibiting such structure and function. More particularly, some implementations of the present teachings can provide an orthopedic brace which effectively supports and/or stabilizes a joint on the body of a wearer on which the orthopedic brace is mounted, thereby providing the wearer with a beneficial physiological effect.

In accordance with one approach consistent with the present teachings, an orthopedic brace for a body joint comprises a longitudinal support assembly and a tensioner. The longitudinal support assembly has a first longitudinal support, a second longitudinal support and a hinge joint rotatably connecting the first and second longitudinal supports to one another, thereby rendering the first and second longitudinal supports positionable at different hinge joint angles. The first longitudinal support has a first tensioner anchor and the second longitudinal support has a second tensioner anchor and a cam.

The tensioner is attached to the first and second tensioner anchors and follows a tensioner path extending between the first and second tensioner anchors which includes a tensioner contact segment on the cam. The tensioner has a cam contact segment between the first and second tensioner anchors engaging the tensioner contact segment on the cam. The tensioner applies a variable elastic tensioning force to the first and second longitudinal supports as the first and second longitudinal supports rotate about the hinge joint to the different hinge joint angles.

The first longitudinal support can have a first end and a second end, the second longitudinal support can have a first end and a second end and the tensioner can have a first end, a second end and an intermediate portion between the first and second ends of the tensioner. The first end of the tensioner can be attached to the first tensioner anchor and the second end of the tensioner can be attached to the second tensioner anchor. The hinge joint can rotatably connect the second end of the first longitudinal support and the first end of the second longitudinal support. The cam can be at the first end of the second longitudinal support. The cam contact segment can be on the intermediate portion of the tensioner. The cam contact segment can variably engage the tensioner contact segment at different positions on the cam.

In accordance with one embodiment of the present approach, the tensioner can have a spring on the intermediate portion positioned between the cam and the second tensioner anchor in the tensioner path. The spring can have an elastic tensioning force characteristic. The spring can have a variable length varying as a function of the hinge joint angles and the variable tensioning force can vary as a function of the variable length of the spring. The variable tensioning force also can vary as a function of the different positions of the tensioner contact segment on the cam. In accordance with an alternate embodiment of the present approach, the spring is a first spring and the elastic tensioning force characteristic has a first value. The orthopedic brace further comprises a second spring having the same elastic tensioning force characteristic, but with a second value different from the first value. The second spring can be selectively interchangable with the first spring.

In accordance with another embodiment of the present approach, the tensioner can have a first operating orientation. The variable tensioning force can substantially increase as the hinge joint angle approaches 180° from 90° and substantially decreases as the hinge joint angle approaches 90° from 180°at the first operating orientation of the tensioner.

In accordance with an alternate embodiment of the present approach, the tensioner can be selectively transitionable from the first operating orientation to a second operating orientation. The variable tensioning force can substantially increase as the hinge joint angle approaches 90° from 180° and can substantially decrease as the hinge joint angle approaches 180° from 90° when the tensioner is in the second operating orientation. The second operating orientation of the tensioner can be in essence a reverse of the first operating orientation of the tensioner.

In accordance with another approach consistent with the present teachings, an orthopedic brace for a body joint comprises a first longitudinal support, a second longitudinal support and a hinge joint. The first longitudinal support has a first end, a second end and a first tensioner anchor. The second longitudinal support has a first end, a second end, a second tensioner anchor and a cam at the first end of the second longitudinal support. The cam has a tensioner contact segment. The hinge joint rotatably connects the first and second longitudinal supports to one another at the second end of the first longitudinal support and the first end of the second longitudinal support, thereby rendering the first and second longitudinal supports positionable at different hinge joint angles.

The orthopedic brace further comprises a tensioner having a first end, a second end, an intermediate portion between the first and second ends and a cam contact segment on the intermediate portion. The first end of the tensioner is attached to the first tensioner anchor and the second end of the tensioner is attached to the second tensioner anchor. The cam contact segment engages the tensioner contact segment. The tensioner applies a variable elastic tensioning force to the first and second longitudinal supports as the first and second longitudinal supports rotate about the hinge joint to the different hinge joint angles. The variable elastic tensioning force biases the longitudinal supports toward a desired position corresponding to a desired hinge joint angle.

In accordance with one embodiment of the present approach, the desired hip joint angle is about 180°. In accordance with another embodiment of the present approach, the desired hip joint angle is about 90°.

In accordance with another embodiment of the present approach, the tensioner can have a spring positioned on the intermediate portion between the cam contact segment and the second end of the tensioner and the spring can have an elastic tensioning force characteristic. In accordance with another embodiment of the present approach, the spring can be a first spring and the elastic tensioning force characteristic can have a first value. The orthopedic brace can further comprise a second spring having the same elastic tensioning force characteristic, but with a second value different from the first value. The second spring can be interchangably positioned on the tensioner in place of the first spring.

In accordance with another approach consistent with the present teachings, an orthopedic brace for a body joint comprises a first longitudinal support, a second longitudinal support and a hinge joint rotatably connecting the first and second longitudinal supports to one another, thereby rendering the first and second longitudinal supports positionable at different hinge joint angles. The first longitudinal support has a first tensioner anchor and the second longitudinal support has a second tensioner anchor and a cam including a tensioner contact segment.

The orthopedic brace further comprises a tensioner attached to the first and second tensioner anchors. The tensioner has a cam contact segment positioned between the first and second tensioner anchors engaging the tensioner contact segment on the cam and a spring positioned between the cam contact segment and the second tensioner anchor. The spring applies a variable elastic tensioning force to the first and second longitudinal supports as a function of a spring expansion length when the first and second longitudinal supports rotate about the hinge joint to the different hinge joint angles.

In accordance with one embodiment of the present approach, the spring expansion length can increase when the first and second longitudinal supports approach a hip joint angle of about 180° from a hip joint angle of about 90° and the spring expansion length can decrease when the first and second longitudinal supports approach a hip joint angle of about 90° from a hip joint angle of about 180°. In accordance with another embodiment of the present approach, the spring expansion length increases when the first and second longitudinal supports approach a hip joint angle of about 90°.

The present teachings will be further understood from the drawings and the following detailed description.

### BRIEF DESCRIPTION OF THE DRAWINGS

The below-listed drawing figures illustrate one or more embodiments of the present teachings by way of example and not by way of limitation. Common reference characters may be used among the different drawing figures to indicate the same or similar structural elements.
FIG. 1 is a front view of a hip brace mounted on the left side of a body of a wearer while the wearer is standing. The hip brace of FIG. 1 is an example embodiment of an orthopedic brace.
FIG. 2 is a rear view of the hip brace of FIG. 1 mounted on the left side of the body of the wearer while the wearer is standing.
FIG. 3 is a partial cross section of the medial side of the hip brace of FIG. 1 showing details of a tensioning assembly having utility therein. The medial side of the hip brace is the side which faces the body of the wearer. The hip brace of the present FIG. is mounted on the left side of the body while the wearer is in a seated position with the hip joint in flexion and the tensioning assembly in a relaxed position.
FIG. 4 is a partial cross section of the medial side of the hip brace of FIG. 1 showing details of the tensioning assembly of FIG. 3, but with the wearer lying in a prone position on the back with the hip joint in extension and the tensioning assembly in a tensioned position.
FIG. 5 is a partial cross section of the medial side of the hip brace of FIG. 1 showing details of the tensioning assembly which is reconfigured from the tensioning assembly of FIGS 3. and 4 so that the tensioning assembly is in a tensioned position when the wearer is in a seated position with the hip joint in flexion.
FIG. 6 is a partial cross section of the medial side of the hip brace of FIG. 1 showing details of the reconfigured tensioning assembly of FIG. 5, but with the wearer lying in a prone position on the back with the hip joint in extension and the tensioning assembly in a relaxed position.

### DETAILED DESCRIPTION

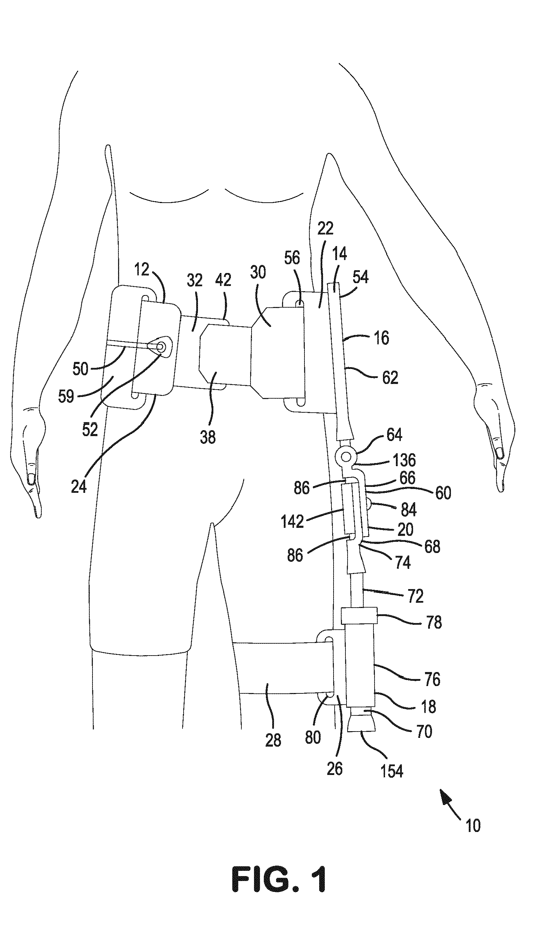

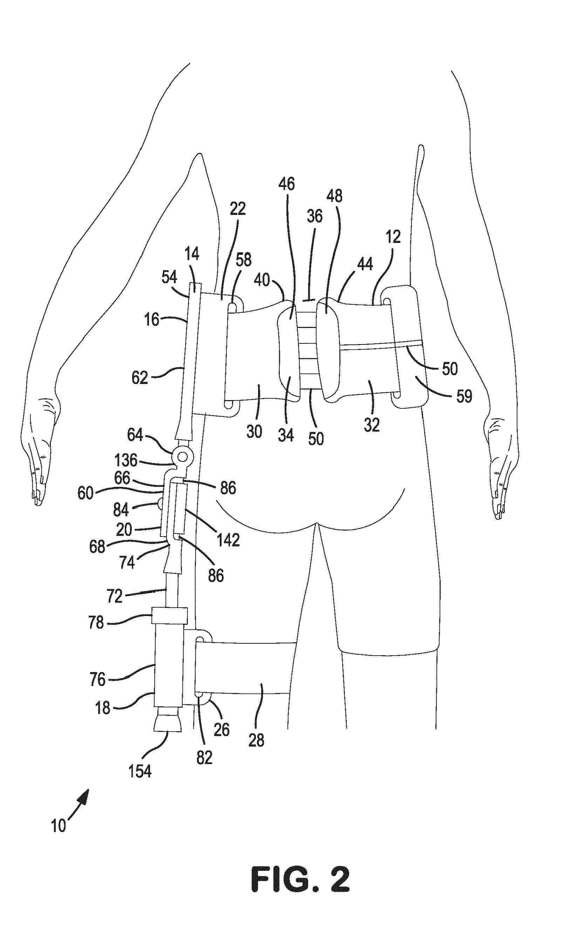

The present teachings present an orthopedic brace mountable on the body of a wearer in the region of an affected joint to generate a variable elastic tensioning force during range of motion activity of the joint, thereby achieving a beneficial physiological effect on the wearer, and more particularly, enhancing the performance of the joint. An example embodiment of an orthopedic brace of the present teachings, which is described in detail hereafter, is a hip brace adapted for mounting on the body of a wearer in the region of an affected hip joint. The hip brace is shown and generally designated 10 with initial reference to FIGS. 1 and 2. The hip brace 10 of this example is comprised of a brace mounting assembly 12 and a longitudinal support assembly 14.

The hip brace 10 is configured so that the longitudinal support assembly 14 is positioned on the same side of the body of the wearer as the affected hip joint when the hip brace 10 is mounted on and secured to the body to support and stabilize the affected hip joint. In the present example embodiment, the affected hip joint is the left hip joint which is on the left lateral side of the body at the junction of the wearer's left leg and torso. Accordingly, the hip brace 10 is configured so that the longitudinal support assembly 14 is likewise positioned on the left lateral side of the body. However, it is readily apparent to one of ordinary skill in the art that if the affected hip joint is instead the right hip joint on the right lateral side of the body at the junction of the wearer's right leg and torso, the hip brace 10 is alternatively configurable so that the longitudinal support assembly 14 can be correspondingly positioned on the right lateral side of the body. Both the left-side and right-side alternate configurations of the hip brace 10 are within the scope of the present teachings.

The longitudinal support assembly 14 is comprised of an upper longitudinal support 16, a lower longitudinal support 18 and a hinge joint 20. The upper and lower longitudinal supports 16, 18 are dynamically connected to one another across the hinge joint 20. The hinge joint 20 functions as a connective link which enables the lower longitudinal support 18 to rotate relative to the upper longitudinal support 16 in correspondence with range of motion activity of the affected hip joint. The upper and lower longitudinal supports 16, 18 and hinge joint 20 are can be formed from one or more substantially rigid materials, such that the upper and lower longitudinal supports 16, 18 and hinge joint 20 themselves exhibit a substantially rigid character.

The term "substantially rigid", as used herein unless expressly stated otherwise, is inclusive of the terms "rigid" and "semi-rigid" and refers to elements or materials in the hip brace 10 which have a high degree of resistance to deformation. As such, "substantially rigid" elements or materials may be in essence completely resistant to elastic deformation or may undergo a only limited degree of elastic deformation in response to routine external forces experienced by the body during activity. Example substantially rigid materials having utility herein include plastics, reinforced plastics, metals, resins, composites and the like. In contrast, "substantially pliant" elements or materials are significantly more pliable and/or more elastically deformable than their "substantially rigid" counterparts. Example substantially pliant materials having utility herein include cloths, laminates, solid foams, leather, and the like.

The brace mounting assembly 12 generally includes one or more cuffs and one or more cooperative straps which are all configured to facilitate mounting and securing the hip brace 10 on the body of the wearer. In accordance with the present example embodiment, the brace mounting assembly 12 can be comprised of a first cuff 22 termed a waist cuff, a corresponding cooperative first strap 24 termed a waist strap, a second cuff 26 termed a leg cuff and a corresponding cooperative second strap 28 termed a leg strap. Both cuffs 22, 26 are generally substantially rigid and both straps 24, 28 are generally substantially pliant.

Referring initially to the waist strap 24, a specific type of waist strap having utility in the present hip brace 10 is commonly termed a belt and is more specifically termed a lumbar support belt. Details of the belt 24 are described hereafter by way of illustration rather by way of limitation. The belt 24 is configured and sized to extend and wrap around the waist and lower torso including the lumbar region of the wearer. As such, the belt 24 substantially encloses the waist and lower torso when the hip brace 10 is properly mounted on the body of the wearer. The belt 24 is comprised of a first belt segment 30, a second belt segment 32 and a mechanical advantage tensioning device 34. The first and second belt segments 30, 32 are separated from one another on the belt 24 by a gap 36 between them, but are connected to one another across the gap 36 by the mechanical advantage tensioning device 34.

The first and second belt segments 30, 32 each can have substantially the same size and shape as the other. As such, the first belt segment 30 has two ends, namely, an attachment end 38 and an adjustment end 40, and the second belt segment 32 similarly has an attachment end 42 and an adjustment end 44. The adjustment ends 40, 44 reside on the posterior of the belt 24, which corresponds to the posterior of the body, when the belt 24 is mounted on the body of a wearer. Conversely, the attachment ends 38, 42 reside on the anterior of the belt 24, which corresponds to the anterior of the body.

The first belt segment 30 and the second belt segment 32 are both fabricated from one or more substantially pliant materials, thereby rendering the first and second belt segments 30, 32 themselves and the belt 24 generally substantially pliant. The material of the belt segments 30, 32 in the present example is also generally or in essence non-stretchable, at least in the circular direction extending around the circumference of the waist of the wearer, thereby rendering the first and second belt segments 30, 32 themselves and the belt 24 generally in essence non-stretchable in the circular direction.

In one example embodiment, the adjustment ends 40, 44 of the first and second belt segments 30, 32, respectively, are formed from a first belt material which is a cloth/foam/cloth laminate and the attachment ends 38, 42 are formed from a second belt material, which is a cloth. The cloth of both the first and second belt materials can have a nappy surface which can function as a loop component of a selectively releasable hook and loop fastener (sold under the trade name VELCRO™). As such, a corresponding hook component of the hook and loop fastener can be releasably attached to any point on the inner or outer face of the first and second belt segments 30, 32 which is cloth-covered.

The added foam layer of the first belt material renders the posterior of the belt 24 generally thicker and less pliant (i.e., stiffer) than the anterior of the belt 24. The posterior of the belt 24 can also be configured with a taller profile than the anterior of the belt 24, in the manner of a conventional weightlifting belt. As a result, the belt 24 having such a shape may cause the wearer to be less flexible about the waist in the backward direction than in the forward direction. It is noted that the second belt material of the belt 24 can also be used as the pliant material of the leg strap 28 since the leg strap 28 of the present example is generally more pliant than the belt 24, or at least more pliant than the posterior of the belt 24.

Although not shown, the posterior of the belt 24 which is thicker and less breathable than the anterior may have a plurality of small openings formed therethrough for ventilation. The thicker posterior may also have a plurality of spaced-apart grooves (not shown) formed therein. The grooves are aligned in correspondence with the longitudinal axis of the wearer's body to provide the stiffened posterior with an articulate construction which advantageously facilitates conformance of the belt 24 to the circumferential arcuate contours of the wearer's body. It is further within the scope of the present teachings to integrate one or more substantially rigid reinforcing elements (not shown) such as plates, stays or the like into the first and/or second belt segments 30, 32 or to externally attach them to the first and/or second belt segments 30, 32 in a manner well known to one of ordinary skill in the art. Any optional reinforcing elements added to the belt 24 may usefully enhance the support function thereof.

The mechanical advantage tensioning device 34 is comprised of a first housing 46, a second housing 48 and a tensioning line 50. The first and second housings 46, 48 are positioned on either side of the gap 36 in the assembled belt 24. In particular, the first housing 46 can be mounted on the first belt segment 30 proximal to the adjustment end 40 thereof and the second housing 48 can be mounted on the second belt segment 32 proximal to the adjustment end 44 thereof. As such, the first belt segment 30 functions as an effective support base for the first housing 46 and the second belt segment 32 functions as an effective support base for the second housing 48. The first and second housings 46, 48 can be formed from a substantially rigid material which is less pliant than the material of the first and second belt segments 30, 32. Mounting of the housings 46, 48 on the belt segments 30, 32, respectively, may be effected by substantially permanent attachment of the housings 46, 48 to the belt segments 30, 32 using permanent attachment means such as riveting, gluing, welding, sewing, stapling, screwing, or the like. Alternatively, mounting may be effected by selective releasable attachment using releasable attachment means such as hook and loop fasteners or the like. The tensioning line 50 can be a relatively thin (i.e., small diameter), light-weight, highly-pliant, high-strength, wear-resistant, and friction-resistant line which can be relatively non-stretchable and which can also be even more pliant than the first and second belt segments 30, 32.

The present belt configuration enables the user to cinch the belt 24 on the body of the wearer by grasping the attachment ends 38, 42 and posteriorly positioning the adjustment ends 40, 44 against the lower back of the wearer while spaced apart from one another. The gap 36 between the adjustment ends 40, 44 is bridged by the mechanical advantage tensioning device 34 which connects the adjustment ends 40, 44 to one another, even while the mechanical advantage tensioning device 34 can remain in a relaxed state. The user manually wraps the length of the second belt segment 32 anteriorly around one side of the wearer's waist and pulls the attachment end 42 tight, anteriorly positioning it over the wearer's abdomen. The user likewise manually wraps the length of the first belt segment 30 anteriorly around the other side of the wearer's waist and pulls the attachment end 38 tight, anteriorly positioning it over the wearer's abdomen in overlapping relation to the attachment end 42 of the second belt segment 32. The overlapping attachment end 38 of the first belt segment 30 has a patch of hook component of a hook and loop fastener on its inner face (not shown). The user releasably fastens the attachment end 38 of the first belt segment 30 to the loop component on the outer face of the second belt segment 32 which the attachment end 38 overlaps, thereby cinching the belt 24 on the body of the wearer. It is further understood that although a hook and loop fastener is described above as one example of a releasable fastening means for cinching the belt 24 on the body, other releasable fasteners have utility herein such as buckles, zippers, buttons, laces and the like and all fall within the scope of the present teachings.

Once the belt 24 is cinched on the body of the wearer, the belt 24 can be further adjustably tensioned by means of the mechanical advantage tensioning device 34 before the wearer fully secures the longitudinal support assembly 14 to the body. The tensioning line 50 has two internal ends (not shown) which attach internally to the housings 46, 48. The path of the tensioning line 50 generally extends from each of its internal ends back and forth between the two housings 46, 48 and within the interiors of the two housings 46, 48. The midsection of the tensioning line 50 exits the interior of the second housing 48 on the lateral side thereof and loops through a tensioning handle 52.

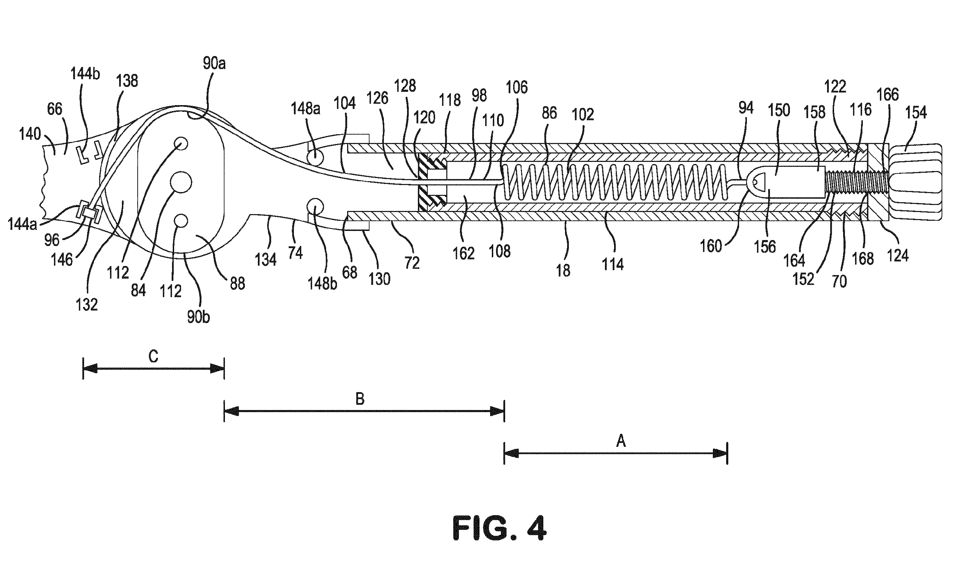

The tensioning line 50 is tensioned by pulling the tensioning handle 52 laterally, which draws the tensioning line 50 further out of the interior of the second housing 48. Conversely, the tensioning line 50 is relaxed by releasing the tensioning handle 52 and allowing the tensioning line 50 to be drawn back into the interior of the second housing 48. Tensioning the tensioning line 50 draws the opposing first and second housings 46, 48 closer together, thereby drawing the adjustment ends 40, 44 of the underlying belt 24 closer together and causing the belt 24 to fit more snugly around the waist. The specifics of an example mechanical advantage tensioning device which is adaptable in accordance with the teaching herein for use in the belt 24 are shown and described in detail in U.S. Patent Application Publication No. 2014/0276305 A1, which is incorporated herein by reference. (See the structure designated by reference no. 16 in FIGS. 2 and 4 of the above-referenced publication and the description of the same structure in the accompanying portions of the specification.)

The waist cuff 22 of the present example functions as a connective link between the belt 24 and the upper longitudinal support 16. As such, the waist cuff 22 engages and attaches to both the belt 24 and the upper longitudinal support 16. In particular, the waist cuff 22 engages and attaches to the upper longitudinal support 16 at one or more points proximal to a first end of the upper longitudinal support 16 termed an upper end 54. Fixable attachment of the waist cuff 22 to the upper longitudinal support 16 can be effected by means of conventional fasteners, such as bolts, rivets, screws or the like. The waist cuff 22 desirably facilitates a close fit and secure mounting of the hip brace 10 on the body of the wearer without substantial movement of the waist cuff 22 relative to the body during routine activity of the wearer. When the hip brace 10 is mounted on the body, the waist cuff 22 is positioned between the body and longitudinal support assembly 14 and correspondingly engages the waist and lower torso immediately above the affected hip joint. The waist cuff 22 generally has a broad plate-like structure resembling a curved plane with a smooth arcuate inner surface contour to generally conform to the curved contour of the side of the waist, thereby continuously engaging a broad area of the wearer's lower torso.

Slidable attachment of the belt 24 to the waist cuff 22 is in the present example effected by threading the belt 24 through an anterior strap slit 56 and a posterior strap slit 58 provided in the waist cuff 22. As such the waist cuff 22 cooperatively connects the belt 24 and longitudinal support assembly 14 and functions in combination with the belt 24 as a first or upper anchor of the longitudinal support assembly 14 to the body of the wearer at a position above the affected hip joint. In addition to its anchoring function, the belt 24 beneficially provides the ancillary function of supporting the lumbar region of the wearer.

The waist cuff 22 of the present example is formed from one or more substantially rigid materials. Due to the substantially rigid character of the waist cuff 22, the inner surface of the waist cuff 22 can be provided with a pad (not shown) which intervenes between the inner surface of the waist cuff 22 and the waist of the wearer to cushion the wearer from the relatively hard inner surface of the waist cuff 22 when the hip brace 10 is mounted on the body of the wearer. The pad is formed from a relatively soft, substantially pliant material such as a foam or the like and is can be attached to the inner surface of the waist cuff 22 by means of a hook and loop fastener or the like (not shown).

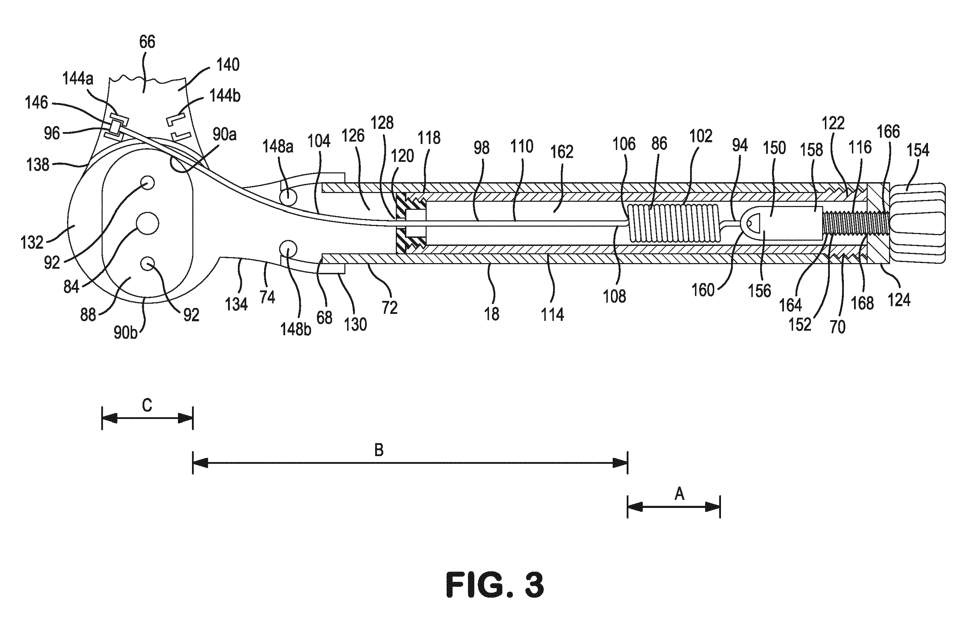

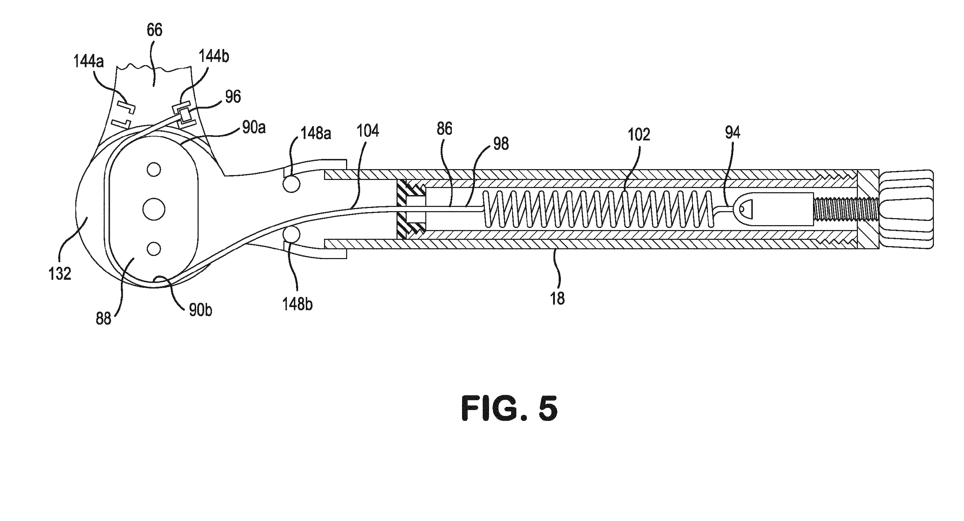

The waist cuff 22 may additionally be fitted with one or more belt locks (not shown) which hold the belt 24 in place within the strap slits 56, 58 to prevent further slidable displacement of the belt 24 relative to the waist cuff 22 once the belt 24 and waist cuff 22 are in their desired relative positions. An example belt lock is a flap which is rotatably attached to the waist cuff 22. The example belt lock typically has a hook component of a hook and loop fastener on its inner face which enables the belt lock to releasably lock the belt 24 into place against the waist cuff 22 when the belt lock is rotated down onto the loop component on the outer surface of the belt 24. A belt lock of this type is shown and described in detail in U.S. Patent Application Publication No. 2014/0276311 A1, which is incorporated herein by reference. (See the structure designated by reference no. 86 in FIGS. 3 and 5 of the above-referenced publication and the description of the same structure in the accompanying portions of the specification.)

The brace mounting assembly 12 of the present example can further comprise a second waist cuff 59 termed an opposing waist cuff to counterbalance the waist cuff 22. The opposing waist cuff 59 has substantially the same configuration and construction as the waist cuff 22 and engages the waist and lower torso on the side of the body opposite the affected hip joint. The opposing waist cuff 59 is provided with strap slits which are in essence the same as described above with respect to the waist cuff 22 and similarly enable slidable attachment of the belt 24 to the opposing waist cuff 59 on the opposite side of the body from the waist cuff 22. However, since the opposing waist cuff 59 neither engages nor attaches directly to the longitudinal support assembly 14, the opposing waist cuff 59 need not be provided with means for attaching the opposing waist cuff 59 to the upper longitudinal support 16 in the manner of the waist cuff 22.

Although the two-segment belt 24 including the mechanical advantage tensioning device 34, which is described above by way of example, has utility in the present hip brace 10, it is readily apparent to the skilled artisan that other known waist straps may alternatively be adaptable in accordance with the teaching herein for use in the hip brace 10. As such, it is understood that such other adapted waist strap structures are likewise within the scope of the present teachings and substantially perform in cooperation with other associated elements of the brace mounting assembly 12 to achieve the same desired function of mounting and securing the longitudinal support assembly 14 to the waist and/or torso of the wearer as described hereafter. For example, an alternate waist strap (not shown), which can be substituted for the belt 24 in the hip brace 10, omits the mechanical advantage tensioning device 34. In accordance with one such alternate embodiment, the alternate belt has a continuous unitary configuration common to most conventional belts rather than the two-segment configuration of the belt 24. The alternate unitary belt is attached to the waist cuff 22 and opposing waist cuff 59 by threading the alternate belt through the strap slits in the cuffs 22, 59 in the same manner as described above with respect to the two-segment configuration.

The longitudinal support assembly 14 of the present example extends substantially downwardly from the waist cuff 22 along the affected hip joint and lateral side (outside) of the leg when the hip brace 10 is securely mounted on the body of the wearer. The longitudinal axis of the longitudinal support assembly 14, and more particularly the longitudinal axis of the upper longitudinal support 16, is aligned substantially perpendicularly at a right angle to the longitudinal axis of the belt 24 when the hip brace 10 is securely mounted on the body of the wearer. As such, the upper longitudinal support 16 extends downward from the waist cuff 22 toward the hinge joint 20 in alignment with the longitudinal axis of the wearer's torso. The upper longitudinal support 16 has a second end termed a lower end 60, which is positioned more proximal to the hinge joint 20 than the more distal upper end 54.

An example construction of the upper longitudinal support 16 includes an upper support member 62, a swivel joint 64 and an upper hinge connector 66. The upper end of the upper support member 62 corresponds identically to the upper end 54 of the upper longitudinal support 16 while the upper hinge connector 66 corresponds identically to the lower end 60 of the upper longitudinal support 16. The upper support member 62 has an unobtrusive flattened profile along most of its length, but transitions to a round profile at its lower end. The lower end is open and hollow to slidably receive the smaller diameter, tubular-shaped upper end of the swivel joint 64 into its interior, thereby effecting attachment of the swivel joint 64 to the upper support member 62. The upper hinge connector 66 is fitted to the lower end of the swivel joint 64 and attaches to the hinge joint 20 to enable rotatable connection of the lower end 60 of the upper longitudinal support 16 to the hinge joint 20. The upper hinge connector 66 may be integrally formed with the lower end of the swivel joint 64, or may alternatively be a separate element from the lower end which is substantially permanently attached thereto by glue or weld bonding or the like, or may alternatively be a separate element which is releasably attached to the lower end by screw threads or the like. The swivel joint 64 enables the user to adjust the relative angle between the upper support member 62 and the hinge joint 20. In accordance with an alternate embodiment (not shown), the swivel joint 64 is omitted from upper longitudinal support 16 and the upper support member 62 and upper hinge connector 66 are attached directly to one another.

The position of the hinge joint 20 relative to the body is immediately adjacent to the affected hip joint. The lower longitudinal support 18 extends downward from the hinge joint 20 and the affected hip joint along the left lateral side of the wearer's thigh in alignment with the longitudinal axis of the wearer's thigh. The lower longitudinal support 18 has a first end termed an upper end 68, which is positioned more proximal to the hinge joint 20, and a second end termed a lower end 70, which is positioned more distal from the hinge joint 20 at a point adjacent to the thigh above the knee and below the affected hip joint. An example construction of the lower longitudinal support 18 includes a lower support member 72, a lower hinge connector 74 and a mounting module 76. The lower end of the lower support member 72 corresponds identically to the lower end 70 of the lower longitudinal support 18 while the lower hinge connector 74 corresponds identically to the upper end 68 of the lower longitudinal support 18.

The lower support member 72 can have a tubular configuration with a substantially hollow interior. The lower hinge connector 74 is fitted to the upper end of the lower support member 72 and attaches to the hinge joint 20 to facilitate rotatable connection of the upper end 68 to the hinge joint 20. The lower end of the lower hinge connector 74 is open to slidably receive the smaller diameter, tubular-shaped upper end of the lower support member 72, thereby effecting attachment of the lower hinge connector 74 to the lower support member 72. The lower hinge connector 74 correspondingly attaches to the hinge joint 20 to enable rotatable connection of the upper end 68 of the lower longitudinal support 18 to the hinge joint 20. The lower hinge connector 74 may be substantially permanently attached to the lower support member 72 by glue or weld bonding or the like or may be releasably attached by screw threads or the like. Alternatively the lower hinge connector 74 may be integrally formed with the upper end of the lower support member 72.

The mounting module 76 is configured as an open tubular sleeve which is shaped and sized to slidably receive the smaller diameter, tubular-shaped lower end of the lower support member 72 in the hollow interior of the mounting module 76, thereby effecting slidable attachment of the mounting module 76 to the lower support member 72. The length of the lower longitudinal support 18 is in the present example adjustable by slidably telescoping the lower support member 72 in or out of the mounting module 76 to shorten or lengthen the lower longitudinal support 18, respectively. The mounting module 76 includes a support member lock 78 which is rotatably attached to the mounting module 76. The support member lock 78 releasably locks the lower support member 72 into place within the interior of the mounting module 76 at a desired overall length of the lower longitudinal support 18 when the support member lock 78 is rotated in a first direction to impose a compressive locking force against the surface of the lower support member 72. The lower support member 72 is unlocked for readjusting the length of the lower longitudinal support 18 by rotating the support member lock 78 in an opposite second direction to release the compressive locking force against the surface of the lower support member 72.

Although not shown, it is also within the scope of the present teachings to provide the upper longitudinal support 16 with similar means for adjusting the length of the upper longitudinal support 16. Alternatively, the upper longitudinal support 16 and/or lower longitudinal support 18 can be provided with means for adjusting the distance between the hinge joint 20 and the waist cuff 22 and/or the leg cuff 26 to achieve in essence the same objective as adjusting the length of the upper longitudinal support 16 and/or lower longitudinal support 18, i.e., adapting the hip brace 10 to different body sizes. In the case of the upper longitudinal support 16, example means for adjusting the distance between the hinge joint 20 and the waist cuff 22 can be the inclusion of multiple alternate selectable attachment points on the upper support member 62 and/or the waist cuff 22 for attaching them to one another. In the case of the lower longitudinal support 18, the mounting module 76 can be omitted altogether. In the absence of the mounting module, the lower support member 72 and leg cuff 26 can be similarly attached directly to one another with the inclusion of multiple alternate selectable attachment points on the lower support member 72 and/or leg cuff 26 for adjusting the distance between the hinge joint 20 and the leg cuff 26.

The leg cuff 26 is positioned between the body and lower longitudinal support 18 and correspondingly engages the lower thigh of the wearer when the hip brace 10 is mounted on the body. The leg cuff 26 also engages and attaches to both the leg strap 28 and the lower longitudinal support 18. More particularly, the leg cuff 26 engages and fixably attaches to the mounting module 76, which correspondingly engages and slidably attaches to the lower support member 72 in a manner described above. Fixable attachment of the leg cuff 26 to the mounting module 76 can be effected by means of conventional fasteners. The leg strap 28 attaches to the leg cuff 26 in substantially the same manner as the belt 24 attaches to the waist cuff 22 by passing through anterior and posterior strap slits 80, 82, respectively, of the leg strap 28. Thus, the leg cuff 26 is able to function as a connective link between the leg strap 28 and the lower longitudinal support 18. The leg cuff 26, leg strap 28 and mounting module 76 function in combination with one another as a second or lower anchor of the longitudinal support assembly 14 to the body of the wearer at a position below the affected hip joint.

The leg cuff 26 of the present example is formed from one or more substantially rigid materials and has a curved-plane structure with a smooth arcuate inner surface contoured to generally conform to the curved contour of the side of the thigh, thereby continuously engaging the area of the wearer's thigh below the affected hip joint. The inner surface of the leg cuff 26 is can be provided with a pad (not shown) which intervenes between the inner surface of the leg cuff 26 and the thigh of the wearer.

The hinge joint 20 in the example embodiment of the present teachings is a monocentric hinge insofar as both the upper and lower longitudinal supports 16, 18 pivot about a single pivot point. The hinge joint 20 of this example has a substantially horizontally-oriented pivot member 84 extending through aligned holes in the upper and lower hinge connectors 66, 74 of the upper and lower longitudinal supports 16, 18, respectively. The pivot member 84 couples the upper and lower hinge connector 66, 74 together in rotatable relation to one another, thereby functioning as a pivot about which the upper and lower longitudinal supports 16, 18 are able to rotate relative to one another. The pivot member 84 can be a cylindrical pin having a solid configuration or a hollowed-out tubular configuration. An example pin having suitability as the pivot member 84 may be selected from among the following hardware: rivets, grommets, bushings, bolts and complementary nuts, combinations thereof, and the like. The upper and lower hinge connectors 66, 74 may be bent or bowed slightly outward from the upper and lower support members 62, 72, respectively, so that the hinge joint 20 does not unduly impinge against the affected hip joint. In sum, the hinge joint 20 rotatably attaches the upper and lower longitudinal supports 16, 18 to one another, thereby enabling rotational displacement of each relative to the other.

Notwithstanding the above, it is understood that the present hinge joint is not limited to any one specific construction. Thus, many known hinges for orthopedic braces, which enable rotation of first and second support members relative to one another, may be adapted to have alternate utility as the hinge joint of the orthopedic brace of the present disclosure, provided such hinges are adapted in accordance with the teaching herein to function in cooperation with a tensioning assembly described hereafter. For example, it is within the scope of the present disclosure to replace the monocentric hinge joint 20 with a polycentric hinge joint, wherein the first and second support members pivot about their own respective pivot points. An example polycentric hinge joint which is adaptable in accordance with the teaching herein for use in combination with the present disclosure is shown and described in detail in U.S. Patent No. 5,772,618, which is incorporated herein by reference. (See the structure designated by reference no. 62b in FIGS. 1 and 8 of the above-referenced publication and the description of the same structure in the accompanying portions of the specification.)

Since the upper and lower longitudinal supports 16, 18 of the hip brace 10 are connected to the torso above the affected hip joint and to the thigh below the affected hip joint, respectively, it is apparent that the upper and lower longitudinal supports 16, 18 rotate relative to one another about the hinge joint 20 during joint range of motion activity in direct correspondence with flexion or extension rotation of the affected hip joint. The degree of rotation that the affected hip joint exhibits is characterized by the hip joint angle, which is the angle defined by the anterior intersection of the thigh and the lower torso at the affected hip joint. The degree of rotation that the upper and lower longitudinal supports 16, 18 exhibit about the hinge joint 20 is correspondingly characterized by the hinge joint angle of the hip brace 10, which is defined by the anterior intersection of the upper and lower longitudinal supports 16, 18 at the hinge joint 20.

The affected hip joint is bent during flexion by rotating it clockwise in a forward and upward direction (i.e., anteriorly) to converge the thigh and lower torso, thereby decreasing the hip joint angle. Since the hinge joint angle in essence identically tracks the hip joint angle, the hinge joint angle correspondingly decreases during flexion of the hip joint in response to convergence of the upper and lower longitudinal supports 16, 18. Conversely, the affected hip joint is straightened during extension by rotating it counterclockwise in a rearward and downward direction (i.e., posteriorly) to diverge the thigh and lower torso, thereby increasing the hip joint angle. The hinge joint angle also correspondingly increases during extension of the hip joint in response to divergence of the upper and lower longitudinal supports 16, 18. If the hinge joint 20 permits the upper and lower longitudinal supports 16, 18 to freely rotate over the full range of a circle (i.e., 0° to 360°) when the hip brace 10 is unmounted on the body, it is apparent that the hinge joint angle will only be limited by the actual physical range of motion of the affected hip joint when the hip brace 10 is mounted on the body. However, it is further apparent that if the hinge joint 20 is provided with means to limit the rotation range of the upper and lower longitudinal supports 16, 18 to less than that of the actual physical range of motion of the affected hip joint, the user can employ these means to desirably limit the range of motion of the affected hip joint for a beneficial therapeutic effect.

Accordingly, it is further within the scope of the present teachings to optionally include means within the hinge joint 20 for selectively adjustably setting hinge rotation limits. As such, a hinge joint having utility in the present hip brace 10 may be further comprised of a selectively adjustable flexion rotation limiter and a selectively adjustable extension rotation limiter (not shown), which function in cooperation with the upper and lower hinge connectors 66, 74, to define flexion and extension rotation limits for the hinge joint and correspondingly for the affected hip joint.

The adjustable flexion rotation limiter enables the user to limit the range of hip joint flexion by selectively setting the flexion rotation limiter at a desired flexion limiting position for the hip joint angle. A typical range of hip joint angles within which the user can selectively set the flexion rotation limiter is a hip joint angle range between about 60° and 180° for flexion. It is noted that the hip joint is at a neutral position when the hip joint angle is 180°, e.g., when the subject is in a fully standing or fully prone position. The hip joint angle is about 90° when the subject is in a normal seated position. The adjustable extension rotation limiter similarly enables the user to limit the range of hip joint extension by selectively setting the extension rotation limiter at a desired extension limiting position for the hip joint angle. A typical range of hip joint angles within which the user can selectively set the extension rotation limiter is a hip joint angle range between about 110° and 190° for extension. It is also within the scope of the present teachings to provide the hinge joint with a releasable hinge rotation lock which enables the user to releasably fix the affected hip joint at a single hip joint angle by selectively manually activating the hinge lock when the hip joint is flexed or extended to the desired hip joint angle.

The structure and function of hinge rotation limiters and locks for orthopedic braces are well known in the art. Details of example adjustable flexion and extension rotation limiters and a hinge rotation lock which may be adaptable in accordance with the teaching herein for use in combination the present disclosure are shown and described in detail in U.S. Patent No. 7,235,059, which is incorporated herein by reference. (See the structure designated by reference nos. 92, 93 and 88, respectively, in FIGS. 1, 2a and 2b of the above-referenced publication and the description of the same structure in the accompanying portions of the specification.)

The present hip brace 10 of the present example further comprises a tensioning assembly which is structurally and functionally integral with the longitudinal support assembly 14. The tensioning assembly of the present embodiment can be associated with the lower support member 72 and the upper and lower hinge connectors 66, 74 which all cooperatively function with one another to enhance the physiological performance of the affected hip joint during range of motion activity. Although not shown herein, it is understood that it is also within the scope of the present teachings to alternatively associate a substantially similar tensioning assembly with a first or upper support member 62, in place of, or in addition to, the tensioning assembly and associated second or lower support member 72 disclosed herein.

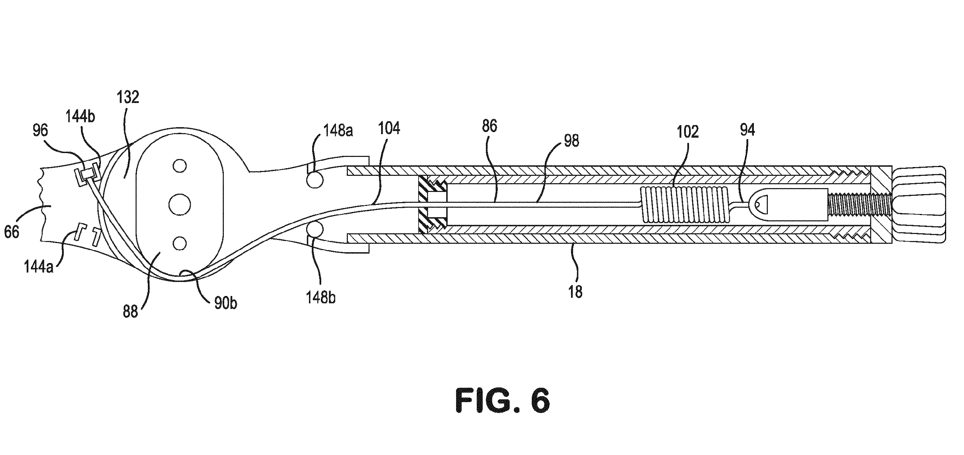

The tensioning assembly of the present example is comprised of a tensioner 86 and a cam 88. In accordance with the embodiment shown in FIGS. 3 and 4, the tensioning assembly, and more particularly the tensioner 86, are selectively configured by the user in a first operating orientation. In accordance with an alternate embodiment shown in FIGS. 5 and 6, the tensioning assembly, and more particularly the tensioner 86, are selectively configured by the user in a second operating orientation. The following description initially describes the tensioning assembly, and more particularly the tensioner 86, configured in the first operating orientation with reference to FIGS. 3 and 4.

The cam 88 is planar-shaped and is structurally an element of both the tensioning assembly and the hinge joint 20. The cam 88 can be formed from a substantially rigid, hard, non-compressible, high-strength, wear-resistant, and friction-resistant material such as a hard plastic or metal. The cam 88 is fixedly mounted to a medial face of the lower hinge connector 74 so that the cam 88 and lower longitudinal support 18, including the lower support member 72 and lower hinge connector 74, are all fixed relative to one another and rotate in unison with one another about the pivot member 84. The cam 88 has a symmetrical oblong configuration which may be partitioned into a first side and a second side. The first and second sides define eccentric, i.e., out-of-round or non-circular, first and second peripheral edges 90a and 90b, respectively, of the cam 88. The cam 88 is mounted to the lower hinge connector 74 by two substantially rigid cam fasteners 92, such as bolts, rivets, screws or the like. The cam 88 is centered on the pivot member 84 and the cam fasteners 92 are offset from the center of the cam 88 and pivot member 84.

Although not shown, the cam fasteners can alternatively be aligned with the center of the cam 88 and pivot member 84 and can be structurally integral with the pivot member 84. In any case, the distance between either first or second peripheral edge 90a, 90b and the center of the cam 88, and correspondingly between either first or second peripheral edge 90a, 90b and the pivot member 84, varies as a function of the specific point on the peripheral edge where the distance is measured due to the eccentricity of the first and second peripheral edges 90a, 90b. Notwithstanding the above, it is noted that only the first peripheral edge 90a is employed when practicing the tensioning assembly in the first operating orientation. Thus, it is within the scope of the present teachings to alternatively provide a cam having an eccentric peripheral edge on only one side of the cam.

The tensioner 86 is an elongate elastic tensioning device which has a first end termed a lower end 94 connected to the lower longitudinal support 18, a second end termed an upper end 96 connected to the upper longitudinal support 16 and an intermediate portion 98 running the length of the tensioner 86 between its lower and upper ends 94, 96. As the tensioner 86 extends from its lower end 94, which is anchored to the lower longitudinal support 18, to its upper end 96, which is anchored to the upper longitudinal support 16, a segment of the intermediate portion 98 of the tensioner 86 termed a cam contact segment variably engages a segment of the first peripheral edge 90a of the cam 88 termed a tensioner contact segment. The actual length of the cam contact segment on the intermediate portion 98 of the tensioner 86 and the length of the tensioner contact segment on the first peripheral edge 90a of the cam 88 vary as a function of the varying hinge joint angle between the upper longitudinal support 16 and lower longitudinal support 18 during joint range of motion activity which is described in greater detail hereafter.

When configured in the first operating orientation, the tensioner contact segment is selectively positioned on the first peripheral edge 90a of the cam 88. As such the first operating orientation is termed an anterior orientation because the tensioner contact segment is more proximal to the anterior side of the body of the wearer when the hip brace 10 is operatively mounted on the body. The tensioner contact segment on the first peripheral edge 90a of the cam 88 effectively functions in the manner of a dynamic fulcrum with respect to the tensioner 86, enabling the tensioner 86 to generate and apply a variable elastic tensioning force to the upper and lower longitudinal supports 16, 18 in a desired direction of their rotation about the hinge joint 20, which biases the upper and lower longitudinal supports 16, 18 toward a desired hinge joint angle. The extent to which the elastic tensioning force (alternately termed the biasing force) biases the upper and lower longitudinal supports 16, 18 is a function of the magnitude of the elastic tensioning force, which in turn is a function of the actual locations of the cam contact segment on the intermediate portion 98 of the tensioner 86 and the tensioner contact segment on the peripheral edge 96 of the cam 88 at any time during joint range of motion activity. It will be apparent from the detailed description of the tensioning assembly which follows that the magnitude of the elastic tensioning force, the relative position of the tensioner 86 and cam 88 to one another and the relative position of the upper and lower longitudinal supports 16, 18 to one another are all inter-correlated.

The tensioner 86 of the present example is comprised of a spring 102 and an extension line 104, which are serially attached to one another at a first end of the spring 102 termed an upper end 106 and a first end of the extension line 104 termed a lower end 108. The second end of the spring 102, termed a lower end, can correspond to the lower end 94 of the tensioner 86. The second end of the extension line 104, termed an upper end, can correspond to the upper end 96 of the tensioner 86. An intermediate portion 110 of the extension line 104, which runs the length of the extension line 104 between its lower and upper ends 108, 96, can correspond in part to the intermediate portion 98 of the tensioner 86.

The spring 102 and extension line 104 may be integrally formed with one another, thereby effecting substantially permanent attachment of the upper end 106 and the lower end 108 to one another in the tensioner 86. Alternatively, the spring 102 and extension line 104 may be separate elements which are substantially permanently attached to one another at their upper and lower ends 106, 108, respectively, by reliable securing means such as crimping, welding, soldering or the like during construction of the tensioner 86.

The spring 102 has a predetermined set of values of its elastic tensioning force characteristics, such as spring constant, elastic limit, elastic modulus and the like, which define the force-generating capabilities of the spring 102. The spring 102 of the present example is an extension spring such as a coil spring having a helical configuration. When a force is applied to an extension spring which expands the length of the spring to a stretched or tensioned state from its shorter unexpanded rest or relaxed state, the spring exerts a counter-force which seeks to return the spring to its rest or relaxed state. Thus, an extension spring is by definition an elastic device which stores mechanical energy when stretched. Although the spring 102 itself is elastically deformable, the spring 102 may typically be formed from a relatively non-stretchable material such as a high-strength metal. The spring 102 itself is also may typically be highly-resistant to inelastic deformation.

The extension line 104 of the present example is a substantially linear, substantially pliant, high-strength, wear-resistant, and friction-resistant monofilament or multi-filament line which is also resistant to inelastic deformation. In the case of a multi-filament line, the filaments may be woven, braided or twisted together. The extension line 104 can be formed from a relatively non-stretchable material such as a high-strength metal and the extension line 104 itself is typically also non-stretchable. Lines satisfying the above criteria and having utility herein are commonly described as cables, cords, wires or the like. In the case, where the spring 102 and extension line 104 have an integral construction as described above, they can be formed from a continuous identical material.

Notwithstanding the above, it is understood that the tensioner 86 is not limited to any one specific construction. Thus, the spring can alternatively have a substantially linear configuration, as in the manner of a bungee cord, which is formed from a stretchable, elastic material. Such an alternate configuration is within the scope of the present disclosure. It is also within the scope of the present disclosure to omit the extension line from the tensioner and replace it in its entirety with a spring which extends the full length of the tensioner. In this case, the upper end of the spring and the upper end of the tensioner correspond to one another.

In accordance with the embodiment shown herein, the tensioning assembly can be further comprised of a spring cartridge 114 and a tension adjuster 116. The spring cartridge 114 is in essence a housing for the tensioner 86 and more particularly for the spring 102. The spring cartridge 114 can be substantially rigid and have a tubular configuration with a hollow interior, within which the entirety of the spring 102 and at least the lower end 108 of the extension line 104 can reside. The spring cartridge 114 is shaped and sized to be slidably received and retained within the hollow interior of the larger diameter, tubular-shaped open lower end 70 of the lower support member 72. The length of the spring cartridge 114 is typically substantially less than the length of the lower support member 72 such that the interior of the lower support member 72 receives and retains the entire length of the spring cartridge 114 therein.

A first end of the spring cartridge 114 termed an upper end 118 has female threads on its inside which are sized in correspondence with male threads on the outside of a first plug termed an upper plug 120 which is screwed into the upper end 118 to cap it. The spring cartridge 114 and associated tensioner 86 are removably assembled in the hip brace 10 by inserting the capped upper end 118 of the spring cartridge 114 into the open lower end 70 of the lower support member 72 and conveying the spring cartridge 114 up into the interior of the lower support member 72 until a second end of the spring cartridge 114 termed a lower end 122 aligns with the lower end 70 of the lower support member 72. The lower end 122 of the spring cartridge 114 has male threads on its outside which terminate at a knurled collar 124 which is attached to a bottom edge of the lower end 122. The male threads are sized in correspondence with female threads on the inside of the lower end 70 of the lower support member 72. Accordingly, the male threads on the lower end 122 are screwed into the female threads on the lower end 70 so that the collar 124 on the lower end 122 of the spring cartridge 114 is in essence flush with the lower end 70 of the lower support member 72 and the spring cartridge 114 is fully nested and removably retained in the interior of the lower support member 72.

As noted above, the relative lengths of the spring cartridge 114 and lower support member 72 can be configured such that the upper end 118 of the spring cartridge 114 does not extend all the way to the upper end 68 of the lower support member 72 when the lower ends 70, 122 are screwed together. As a result, a void space 126 would exist in the interior of the lower support member 72 between the upper end 68 of the lower support member 72 and the capped upper end 118 of the spring cartridge 114. An opening 128 is provided in the upper plug 120 which is sized to permit the tensioner 86, and in some implementations more particularly the upper end 96 and at least some of the intermediate portion 98 of the tensioner 86 (i.e., upper end 96 and intermediate portion 110 of the extension line 104), to exit the interior of the spring cartridge 114 and pass through the void space 126 in the interior of the lower support member 72.

The lower hinge connector 74 of the present example is configured with a first end termed a lower end 130, a second end termed an upper end 132 and a transition body 134 extending between and connecting the lower and upper ends 130, 132 to one another. The elements of the lower hinge connector 74 are typically all substantially rigid. The lower end 130 has a three-sided open configuration which is shaped and sized to slidably receive and retain the smaller diameter, tubular-shaped open upper end 68 of the lower support member 72. The upper end 132 has a round planar plate configuration and is alternatively termed a lower hinge plate which is structurally an element of both the lower longitudinal support 18 and the hinge joint 20. The lower hinge plate 132 is the specific element of the lower hinge connector 74 on which the cam 88 is fixedly mounted. The transition body 134 is configured to structurally transition the lower hinge connector 74 from a three-dimensional configuration to a planar plate configuration. The lower end 130 and transition body 134 in combination provide a pathway for the tensioner 86, and in some implementations more particularly a pathway for the intermediate portion 98 of the tensioner 86 (i.e., intermediate portion 110 of the extension line 104), between the void space 126 in the interior of the lower support member 72 and the tensioner contact segment on the first peripheral edge 90a of the cam 88.

The upper hinge connector 66 is configured with a first end termed an upper end 136 (shown in FIGS. 1 and 2), a second end termed a lower end 138 and a transition body 140 extending between and connecting the upper and lower ends 136, 138 to one another. The elements of the upper hinge connector 66 are typically all substantially rigid. As described above, the upper end 136 of the upper hinge connector 66 is attached to the swivel joint 64 at the lower end of the upper support member 62. The lower end 138 of the upper hinge connector 66 has a round planar plate configuration and is alternatively termed an upper hinge plate which is structurally an element of both the upper longitudinal support 16 and the hinge joint 20.

The upper and lower hinge plates 138, 132 are the specific structures of the upper and lower hinge connectors 66, 74, respectively, which have aligned holes extending therethrough to receive the pivot member 84, thereby enabling the pivot member 84 to couple the upper and lower hinge plates 138, 132 together in rotatable relation to one another and creating the rotatable hinge joint 20. The hinge joint 20 can have a hinge cover 142 (shown in FIGS. 1 and 2) overlying the coupled upper and lower hinge plates 138, 132 and associated cam 88 to shield the inner workings of the hinge joint 20 from external interferences. The hinge joint 20 also can have a thin planar glide washer (not shown) positioned between the abutting faces of the upper and lower hinge plates 138, 132 to reduce friction during hinge joint rotation. The upper and lower hinge plates 138, 132, and in some implementations specifically the lower hinge plate 132, are also the specific structures of the hip brace 10 which are adaptable to accommodate the above-described selectively adjustable flexion and extension rotation limiters and rotation lock of the hinge joint 20 if included therein.

The transition body 140 of the upper hinge connector 66 is configured to structurally transition the upper hinge connector 66 to a planar plate configuration. The upper hinge connector 66 and lower hinge connector 74 differ from one another insofar as the upper hinge connector 66, and in some implementations more particularly the transition body 140 of the upper hinge connector 66, includes a first tensioner anchor termed a first upper tensioner anchor 144a, which is omitted from the lower hinge connector 74. The first upper tensioner anchor 144a anchors the upper end 96 of the tensioner 86, which corresponds to the upper end of the extension line 104, to the upper longitudinal support 16. The first upper tensioner anchor 144a can be a shaped enclosure formed on the face of the transition body 140 of the upper hinge connector 66, which is configured to receive and retain an enlarged anchor nub 146 fixedly attached to the upper end 96 of the tensioner 86. The first upper tensioner anchor 144a has a posterior orientation relative to the body of the wearer in the first operating orientation of the tensioning assembly, and more particularly the tensioner 86, when the hip brace 10 is mounted on the body, i.e., the first upper tensioner anchor 144a is positioned on the hip brace 10 more proximal to the posterior side of the body when the hip brace 10 is mounted on the body.

The upper hinge connector 66 and lower hinge connector 74 further differ from one another insofar as the lower hinge connector 74, and in some implementations more particularly the transition body 134 of the lower hinge connector 74, includes a first guide member 148a, which is omitted from the upper hinge connector 66. The first guide member 148a can have a cylindrical configuration with a smooth curved face formed from a friction reducing material, which is retained in or on the transition body 134. The first guide member 148a can be either a rotatable pulley or a fixed post. The first guide member 148a directs the intermediate portion 98 of the tensioner 86 (i.e., intermediate portion 110 of the extension line 104) as it exits the upper end 68 of the lower support member 72 and extends toward the cam 88. The first guide member 148a has an anterior orientation relative to the body of the wearer in the first operating orientation of the tensioning assembly, and more particularly the tensioner 86, when the hip brace 10 is mounted on the body.

The tension adjuster 116 is positioned at the lower end 122 of the spring cartridge 114, which is correspondingly positioned at the lower end 70 of the lower support member 72. The tension adjuster 116 can be comprised of a tension adjustment base 150, a tension adjustment rod 152 and a tension adjustment knob 154, which are all typically substantially rigid. The tension adjustment base 150 has an upper end 156 and a lower end 158 and is positioned within the interior of the spring cartridge 114 at its lower end 122, which is correspondingly nested within the lower end 70 of the lower support member 72. The tension adjustment base 150 is comprised of a second tensioner anchor termed a lower tensioner anchor 160. The lower tensioner anchor 160 is positioned at the upper end 156 of the tension adjustment base 150 and anchors the lower end 94 of the tensioner 86, which corresponds to the lower end of the spring 102, to the lower longitudinal support 18. The lower tensioner anchor 160 can be a retention loop through which the lower end 94 of the tensioner 86 is threaded and interlocked. The lower tensioner anchor 160 can be configured so that the tension adjustment base 150 is able to rotate independent of the tensioner 86.

The variable distance between the upper end 156 of the tension adjustment base 150 and the upper end 118 of the spring cartridge 114 defines a spring expansion chamber 162 in the interior of the spring cartridge 114 within which the extension spring 102 is able to expand as it is stretched. As such, this distance corresponds to the variable length of the spring expansion chamber 162. An alternate variable length reference is the distance between the upper end 156 of the tension adjustment base 150 and the hinge joint 20.

The tension adjustment rod 152 is a male threaded member having a first end termed an upper end 164, which is fixably attached to the tension adjustment base 150. The tension adjustment rod 152 has a second end termed a lower end 166, which is fixedly attached to the tension adjustment knob 154, thereby connecting the tension adjustment knob 154 and the tension adjustment base 150 to one another. The tension adjustment rod 152 extends from its upper end 164 to the tension adjustment knob 154 through a central longitudinal bore 168 in the collar 124 which is lined with female threads. The tension adjustment knob 154 is exposed to the exterior for manual access by the user.

The tension adjuster 116 enables a user to selectively adjust the length of the spring expansion chamber 162 by selectively manually rotating the tension adjustment knob 154 in either direction of rotation. Rotating the tension adjustment knob 154 in a first direction of rotation, which can be a clockwise direction, screws the tension adjustment rod 152 further into the bore 168, thereby shortening the length of the spring expansion chamber 162. Rotating the tension adjustment knob 154 in a second direction of rotation, which is typically a counterclockwise direction, unscrews the tension adjustment rod 152 further out of the bore 168, thereby increasing the length. Selective adjustment of the length of the spring expansion chamber 162 correspondingly results in selective adjustment of the maximum available elastic tensioning force that the tensioner 86, and in some implementations more particularly the spring 102 housed in the spring cartridge 114, can apply to the upper and lower longitudinal supports 16, 18. The length of the spring expansion chamber 162 and the elastic tensioning force of the tensioner 86 are directly correlated to one another. Therefore, increasing the length of the spring expansion chamber 162 increases the maximum available elastic tensioning force of the tensioner 86 by permitting a longer available distance for the spring 102 to stretch. Decreasing the length decreases the maximum available elastic tensioning force by shortening the available distance for the spring 102 to stretch.

In accordance with an alternate embodiment not shown, the spring cartridge 114 is omitted from the tensioning assembly and the spring 102 is housed directly in the hollow interior of the lower support member 72. The tension adjuster 116 may also be omitted from the tensioning assembly and the lower end 94 of the spring 102 fixably connected to the lower end 70 of the lower longitudinal support 18.

Before initiating operation of the tensioning assembly, the user suitably adjusts the lengths of the lower longitudinal support 18 (and upper longitudinal support 16 if length adjustment means are provided) in correspondence with the size of the wearer and mounts and secures the hip brace 10 on the body in accordance with the above teaching. The wearer then initiates operation of the tensioning assembly by engaging in range of motion activity for the affected hip joint, for example by engaging in normal routine physical activity or by engaging in generalized or specialized range of motion exercise for the affected hip joint, either solo or under the supervision of a healthcare professional.

Operation of the tensioning assembly is described below with continuing reference to FIGS. 3 and 4. FIG. 3 shows the path of the tensioner 86 in the hip brace 10 when the affected hip joint is rotated to a bent flexion position represented by a 90° hip joint angle. The hinge joint 20 is correspondingly rotated to a 90° hinge joint angle, thereby placing the tensioner 86 in a bent relaxed (i.e., untensioned) position. By comparison, FIG. 4 shows the path of the tensioner 86 when the affected hip joint is rotated to a straight extension position represented by a 180° hip joint angle. The hinge joint 20 is correspondingly rotated to a 180° hinge joint angle, thereby placing the tensioner 86 in a straight tensioned position, which is substantially more tensioned and substantially less relaxed than the relaxed position of the tensioner 86. It is noted the hinge cover 142 has been omitted from the hinge joint 20 in FIGS. 3 and 4 for clarity.

The path of the tensioner 86 extends in either case between the lower tensioner anchor 160 and the first upper tensioner anchor 144a. Starting at the lower tensioner anchor 160, the path of the tensioner 86 passes in series through the spring expansion chamber 162 of the spring cartridge 114, the plug opening 128 in the upper plug 120 and the void space 126 in the upper end 68 of the lower support member 72. As the tensioner 86 exits the upper end 68, the first guide member 148a directs the path of the tensioner 86 from the transition body 134 anteriorly over the planar face of the lower hinge plate 132 and into engagement with the cam 88 where the cam contact segment of the tensioner 86 engages the tensioner contact segment on the first peripheral edge 90a of the cam 88. After the cam 88, the path of the tensioner 86 continues anteriorly over the planar face of the lower hinge plate 132 until terminating at the first upper tensioner anchor 144a. It is noted that an opening (not shown) for the tensioner 86 to pass through is also provided in the wall of the hinge cover which is omitted from FIGS. 3 and 4.

In the present embodiment of the hip brace 10, where the tensioner 86 is comprised of the spring 102 and extension line 104, the path of the spring 102 is wholly contained within the interior of the lower support member 72, and in some implementations more particularly wholly contained within the spring expansion chamber 162. The junction of the spring 102 and the extension line 104 at the upper end 106 of the spring 102 and the lower end 108 of the extension line is likewise within the interior of the lower support member 72, and in some implementations more particularly within the spring expansion chamber 162. The path of the extension line 104 corresponds to the remainder of the path of the tensioner 86 recited above, wherein the bulk of the path is occupied by the intermediate portion 110 of the extension line 104 with only the upper terminus of the path being occupied by the upper end 86 of the extension line 104 as it approaches the first upper tensioner anchor 144a.

When the hinge joint angle is 90°, as shown in FIG. 3, the tensioner 86 is in the relaxed position with the extension spring 102 in a relaxed state, which may be alternately described as a rested, untensioned, unstressed, unstretched or unexpanded state. As such, the length of the variable-length spring 102 (approximated by A in FIG. 3) is relatively short. The total length of the tensioner 86, which includes the length of the variable-length spring 102 and the substantially fixed length of the extension line 104, is correspondingly relatively short. A segment of the extension line 104 is termed a pre-cam segment (approximated by B in FIG. 3), which encompasses the lower end 108 of the extension line 104 and the bulk of the intermediate portion 110 of the extension line 104. The pre-cam segment of the extension line 104 resides in the segment of the tensioner path extending from the lower end 108 of the extension line 104 to the beginning of the tensioner contact segment on the cam 88, is relatively long. Conversely, a segment of the extension line 104 is termed a post-cam segment (approximated by C in FIG. 3), which encompasses the upper end 96 of the extension line 104 and only a short length of the intermediate portion 110 of the extension line 104. The post-cam segment of the extension line 104 resides in the segment of the tensioner path extending from the beginning of the tensioner contact segment on the cam 88 to the first upper tensioner anchor 144a, is relatively short.

It is correspondingly apparent that the location of the cam contact segment on the intermediate portion 110 of the extension line 104 is relatively more proximal (i.e., less distal) to the upper end 96 of the extension line 104, while the location of the tensioner contact segment on the peripheral edge 96 of the cam 88 is relatively more proximal to the first upper tensioner anchor 144a. It is additionally noted that the lengths of the cam contact segment and the tensioner contact segment are both relatively short since the tensioner 86, and in some implementations more particularly the extension line 104, only tangentially engage the cam 88.

To transition the hip brace 10 to a hinge joint angle of 180°, as shown in FIG. 4, the affected hip joint is straightened to extension by posteriorly rotating it in a counterclockwise direction, which increases the hip joint angle. Since the hinge joint angle in essence identically tracks the hip joint angle, the hinge joint angle correspondingly increases during extension of the hip joint. Conversely, to transition the hip brace 10 back to a hinge joint angle of 90°, as shown in FIG. 3, the affected hip joint is bent to flexion by anteriorly rotating it in a clockwise direction, which decreases the hip joint angle. The hinge joint angle correspondingly decreases during flexion of the hip joint.

When the hinge joint angle is 180°, the tensioner 86 is in the tensioned position with the extension spring 102 in a tensioned state, which may be alternately described as a stressed, stretched or expanded state. As such, the length A of the spring 102, and correspondingly the total length of the tensioner 86, is relatively long. The length of the pre-cam segment B of the extension line 104 is relatively short, while conversely the length of the post-cam segment C of the extension line 104 is relatively long. It is further apparent that the location of the cam contact segment on the intermediate portion 110 of the extension line 104 is relatively less proximal (i.e., more distal) to the upper end 96 of the extension line 104, while the location of the tensioner contact segment on the peripheral edge 96 of the cam 88 is relatively less proximal to the first upper tensioner anchor 144a. It is additionally noted that the lengths of the cam contact segment and the tensioner contact segment are both relatively long since the tensioner 86, and in some implementations more particularly the extension line 104, wraps around and engages a substantially longer arc of the peripheral edge 96 of the cam 88 as the cam 88 directs the extension line 104 toward the less proximal first upper tensioner anchor 144a.

It is apparent from FIGS. 3 and 4 that as the hip joint angle of the body, and correspondingly the hinge joint angle of the hip brace 10, approaches 180° from 90° during extension rotation of the hip joint and associated hinge joint 20, the cam 88 automatically substantially increases the degree of elastic tensioning force applied to the tensioner 86 on the upper and lower longitudinal supports 16, 18, respectively, in accordance with a non-linear function. As such, the rate of the elastic tensioning force increase is substantially greater at positions more proximal to the hip joint and hinge joint angle of 180° from the direction of 90° and the rate of the increase is substantially lesser at positions more distal from the hip joint and hinge joint angle of 180° in the direction of 90°.

In sum, the increase in the degree of elastic tensioning force is a result of increasing the total length A of the tensioner 86 (and more particularly, increasing the length of the spring 102), which correlates to decreasing the length of the pre-cam segment B and increasing the length of the post-cam segment C of the extension line 104. An increasing degree of elastic tensioning force, also correlates to: 1) an increasing distance between the cam contact segment on the extension line 104 and the upper end 96 of the extension line 104; 2) an increasing distance between the tensioner contact segment on the cam 88 and the first upper tensioner anchor 144a; and 3) increasing lengths of the cam and tensioner contact segments.

Conversely, as the hip joint angle of the body, and correspondingly the hinge joint angle of the hip brace 10, approaches 90° from 180° during flexion rotation of the hip joint and hinge joint 20, the cam 88 automatically substantially decreases the degree of elastic tensioning force applied to the tensioner 86 on the upper and lower longitudinal supports 16, 18, respectively, in accordance with a non-linear function. As such, the rate of the elastic tensioning force decrease is greater at positions more proximal to the hip joint and hinge joint angle of 180° from the direction of 90° and the rate of the decrease is lesser at positions more distal from the hip joint and hinge joint angle of 180° in the direction of 90°.

The decrease in the degree of elastic tensioning force is a result of decreasing the total length A of the tensioner 86 (and more particularly, decreasing the length of the spring 102), which correlates to increasing the length of the pre-cam segment B and decreasing the length of the post-cam segment C of the extension line 104. A decreasing degree of elastic tensioning force, also correlates to: 1) a decreasing distance between the cam contact segment on the extension line 104 and the upper end 96 of the extension line 104; 2) a decreasing distance between the tensioner contact segment on the cam 88 and the first upper tensioner anchor 144a; and 3) decreasing lengths of the cam and tensioner contact segments.

A significant level of potential mechanical energy is stored in the tensioner 86 when the hip joint and hinge joint angle reaches the 180° position of substantially full extension from a lower angular flexion position. This results from the increasing level of elastic tensioning force that the tensioner 86 applies to the upper and lower longitudinal supports 16, 18 as the hip joint and hinge joint angle approaches 180°. The potential mechanical energy stored in the tensioner 86 at 180° hip joint and hinge joint angle desirably applies a flexural loading to the hip brace 10, which biases the hip joint and hinge joint angle back toward the 90° position of flexion and provides the wearer of the hip brace 10 with a beneficial flexion assist when flexing the hip joint.

An ancillary desirable effect of increasing the degree of elastic tensioning force applied by the tensioner 86 to the upper and lower longitudinal supports 16, 18 as the hip joint and hinge joint angle approaches 180° is that the wearer of the hip brace 10 must expend greater effort, i.e., perform more work, to extend the hip joint to a straight extension position than would otherwise be required in the absence of the hip brace 10. Therefore, the wearer desirably strengthens the leg, and more particularly the muscles of the upper leg responsible for extension of the hip joint, by working to overcome the increasing elastic tensioning force applied by the hip brace 10 during extension of the hip joint.

FIGS. 5 and 6 show an alternate embodiment, wherein the tensioning assembly, and more particularly the tensioner 86, are selectively reconfigured from the first operating orientation shown in FIGS. 3 and 4 to the second operating orientation, which is i a reverse of the first operating orientation. When the tensioner 86 is transitioned to the second operating orientation, the tensioner path is reconfigured by diverting and lengthening it to include the second upper tensioning anchor 144b and second guide member 148b, while omitting the first upper tensioning anchor 144a and first guide member 148a from the tensioner path. As such, the second tensioner guide member 148b has a posterior orientation and the second upper tensioner anchor 144b has an anterior orientation in the second operating orientation of the tensioning assembly, and more particularly the tensioner 86, when the hip brace 10 is mounted on the body. It is further noted that an alternate tensioner 86 is substituted in the present alternate embodiment of FIGS. 5 and 6 for the tensioner 86 in the embodiment of FIGS. 3 and 4. The tensioner 86 of FIGS. 5 and 6 has a substantially longer extension line 104 than the tensioner 86 of FIGS. 3 and 4 to accommodate the longer tensioner path in the embodiment of FIGS. 5 and 6.

In accordance with the present alternate embodiment, the tensioner 86 is in the tensioned position with the extension spring in a tensioned state when the hip joint and hinge joint angle is 90° and the tensioner is in the relaxed position with the extension spring in a relaxed state when the hip joint and hinge joint angle is 180°. More particularly, as the hip joint and hinge joint angle approaches 90° from 180° during flexion rotation of the hip joint and associated hinge joint 20, the cam 88 increases the degree of elastic tensioning force applied by the tensioner 86 to the upper and lower longitudinal supports 16, 18, respectively, by increasing the total length A of the tensioner 86, and more particularly, by increasing the length of the spring 102. As the hip joint and hinge joint angle approaches 180° from 90° during extension rotation of the hip joint and associated hinge joint 20, the cam 88 decreases the degree of elastic tensioning force applied by the tensioner 86 to the upper and lower longitudinal supports 16, 18, respectively, by decreasing the total length A of the tensioner 86, and more particularly, by decreasing the length of the spring 102.

In the present alternate embodiment, a significant level of potential mechanical energy is stored in the tensioner 86 when the hip joint and hinge joint angle reaches the 90° position of substantial flexion from a higher angular extension position. This results from the increasing level of elastic tensioning force that the tensioner 86 applies to the upper and lower longitudinal supports 16, 18 as the hip joint and hinge joint angle approaches 90°. The potential mechanical energy stored in the tensioner 86 at 90° hip joint and hinge joint angle desirably applies an extension loading to the hip brace 10, which biases the hip joint and hinge joint angle back toward the 180° position of extension and provides the wearer of the hip brace 10 with a beneficial extension assist when extending the hip joint.

An ancillary desirable effect of increasing the degree of elastic tensioning force applied by the tensioner 86 to the upper and lower longitudinal supports 16, 18 as the hip joint and hinge joint angle approaches 90° is that the wearer of the hip brace 10 must expend greater effort, i.e., perform more work, to flex the hip joint to a bent flexion position than would otherwise be required in the absence of the hip brace 10. Therefore, the wearer desirably strengthens the leg, and more particularly the muscles of the upper leg responsible for flexion of the hip joint, by working to overcome the increasing elastic tensioning force applied by the hip brace 10 during flexion of the hip joint.

In an alternate embodiment, the hip brace 10 is further comprised of one or more interchangable alternate spring cartridges and associated interchangable alternate tensioners (not shown) in addition to the spring cartridge 114 termed the initial first spring cartridge and its associated tensioner 86 termed the initial first tensioner, which have been described in detail above. Each alternate spring cartridge can house its own respective alternate tensioner in substantially the same manner as described above with respect to the first spring cartridge 114 and associated first tensioner 86. In accordance with the present embodiment, the alternate spring cartridges and associated alternate tensioners are interchangable with the first spring cartridge 114 and associated first tensioner 86.

A selected alternate spring cartridge and associated alternate tensioner are interchanged with the initial first spring cartridge 114 and the associated initial first tensioner 86 by removing the first spring cartridge 114 and first tensioner 86 from the hip brace 10 and replacing it with the selected alternate spring cartridge and associated alternate tensioner. More particularly, removal of the first spring cartridge 114 and first tensioner 86 can be effected by withdrawing the anchor nub 146 from the upper tensioner anchor 144, which is the shaped slot in the transition body 140 of the upper hinge connector 66, and unscrewing the lower end 122 of the spring cartridge 114 from the lower end 70 of the lower support member 72. The spring cartridge 114 and the tensioner 86 housed therein are withdrawn from the interior of the lower support member 72 through its open lower end 70 after threading the anchor nub 146 through the transition body 134 of the lower hinge connector 74 and the void space 126 of the lower support member 72. Replacement of the first spring cartridge 114 and associated first tensioner 86 with the selected alternate spring cartridge tensioner and associated alternate tensioner is effected by in essence reversing the above-recited steps of removing the first spring cartridge 114 and associated first tensioner 86.

Each alternate spring cartridge can have a substantially similar construction to the above-described first spring cartridge 114 and each alternate tensioner can have has a substantially similar construction to the above-described first tensioner 86, which in accordance with one of the above-described embodiments includes a spring and an extension line. The alternate extension line of the alternate tensioner may be substantially identical to the extension line 104 of the first tensioner 86. However, the alternate spring of the alternate tensioner can have a different value for one or more of its elastic tensioning force characteristics common with the first spring 102, such as spring constant, than the first spring 102, which renders the alternate tensioner, and more particularly the alternate spring, capable of applying a substantially greater or lesser degree of elastic tensioning force to the upper and lower longitudinal supports 16, 18 than the first tensioner 86, and more particularly the first spring 102.

Accordingly, the user can manually selectively modify the elastic tensioning force application characteristics of the hip brace 10, and more particularly can selectively increase or decrease the elastic tensioning force that the tensioner 86 is capable of applying to the upper and lower longitudinal supports 16, 18: (1) by rotating the tension adjustment knob 154 to selectively adjust the length of the spring expansion chamber 162 in the spring cartridge 114; and/or (2) by replacing the first spring cartridge 114 and first tensioner 86 with a selected alternate spring cartridge and alternate tensioner, thereby substituting an alternate spring having a different value for one or more of its elastic tensioning force characteristics common with the first spring 102, such as spring constant.

Therefore, viewed form a first aspect, there has been described an orthopedic brace for a body joint which has a first longitudinal support, a second longitudinal support and a hinge joint rotatably connecting the longitudinal supports, thereby rendering them positionable at different hinge joint angles. The brace also has a tensioner attached at its opposite ends to the first and second longitudinal supports which intermediately engages a cam positioned at the hinge joint. The tensioner applies a variable elastic tensioning force to the longitudinal supports which varies as a function of the hinge joint angle and biases the longitudinal supports toward a position corresponding to a desired hinge joint angle.

Further examples consistent with the present teachings are set out in the following numbered clauses.
Clause 1. An orthopedic brace for a body joint comprising: a longitudinal support assembly having a first longitudinal support, a second longitudinal support and a hinge joint rotatably connecting said first and second longitudinal supports to one another, thereby rendering said first and second longitudinal supports positionable at different hinge joint angles, wherein said first longitudinal support has a first tensioner anchor and said second longitudinal support has a second tensioner anchor and a cam; and a tensioner attached to said first and second tensioner anchors, wherein said tensioner follows a tensioner path extending between said first and second tensioner anchors and including a tensioner contact segment on said cam, wherein said tensioner has a cam contact segment between said first and second tensioner anchors engaging said tensioner contact segment on said cam, and wherein said tensioner applies a variable elastic tensioning force to said first and second longitudinal supports as said first and second longitudinal supports rotate about said hinge joint to said different hinge joint angles.
Clause 2. The orthopedic brace of clause 1, wherein said first longitudinal support has a first end and a second end, said second longitudinal support has a first end and a second end and said tensioner has a first end, a second end and an intermediate portion between said first and second ends of said tensioner, wherein said first end of said tensioner is attached to said first tensioner anchor, said second end of said tensioner is attached to said second tensioner anchor, wherein said hinge joint rotatably connects said second end of said first longitudinal support and said first end of said second longitudinal support, wherein said cam is at said first end of said second longitudinal support, and wherein said cam contact segment is on said intermediate portion of said tensioner.
Clause 3. The orthopedic brace of clause 2, wherein said tensioner has a spring on said intermediate portion positioned between said cam and said second tensioner anchor in said tensioner path, and wherein said spring has an elastic tensioning force characteristic.
Clause 4. The orthopedic brace of any preceding clause , wherein said cam contact segment on said tensioner variably engages said tensioner contact segment at different positions on said cam.
Clause 5. The orthopedic brace of clause 3 or any clause dependent thereon, wherein said spring has a variable length varying as a function of said hinge joint angles, and wherein said variable tensioning force varies as a function of said variable length of said spring.
Clause 6. The orthopedic brace of any preceding clause, wherein said tensioner has a first operating orientation and said variable tensioning force substantially increases as said hinge joint angle approaches 180° from 90° at said first operating orientation of said tensioner.
Clause 7. The orthopedic brace of clause 6, wherein said variable tensioning force substantially decreases as said hinge joint angle approaches 90° from 180°at said first operating orientation of said tensioner.
Clause 8. The orthopedic brace of clause 6 or 7, wherein said tensioner is selectively transitionable from said first operating orientation to a second operating orientation and said variable tensioning force substantially increases as said hinge joint angle approaches 90° from 180° when said tensioner is in said second operating orientation.
Clause 9. The orthopedic brace of clause 8, wherein said variable tensioning force substantially decreases as said hinge joint angle approaches 180° from 90° at said second operating orientation.
Clause 10. The orthopedic brace of clause 8 or 9, wherein said second operating orientation of said tensioner is in essence a reverse of said first operating orientation of said tensioner.
Clause 11. The orthopedic brace of clause 4 or any clause dependent thereon, wherein said variable tensioning force varies as a function of said different positions of said tensioner contact segment on said cam.
Clause 12. The orthopedic brace of clause 3 or any clause dependent thereon, wherein spring is a first spring and said elastic tensioning force characteristic has a first value, said orthopedic brace further comprising a second spring having said elastic tensioning force characteristic with a second value different from said first value, wherein said second spring is selectively interchangable with said first spring.
Clause 13. An orthopedic brace for a body joint comprising: a first longitudinal support, a second longitudinal support and a hinge joint, wherein said first longitudinal support has a first end, a second end and a first tensioner anchor, wherein said second longitudinal support has a first end, a second end, a second tensioner anchor and a cam at said first end of said second longitudinal support, wherein said cam has a tensioner contact segment, and wherein said hinge joint rotatably connects said first and second longitudinal supports to one another at said second end of said first longitudinal support and said first end of said second longitudinal support, thereby rendering said first and second longitudinal supports positionable at different hinge joint angles; and a tensioner having a first end, a second end, an intermediate portion between said first and second ends and a cam contact segment on said intermediate portion, wherein said first end of said tensioner is attached to said first tensioner anchor and said second end of said tensioner is attached to said second tensioner anchor, wherein said cam contact segment engages said tensioner contact segment, wherein said tensioner applies a variable elastic tensioning force to said first and second longitudinal supports as said first and second longitudinal supports rotate about said hinge joint to said different hinge joint angles, and wherein said variable elastic tensioning force biases the longitudinal supports toward a desired position corresponding to a desired hinge joint angle.
Clause 14. The orthopedic brace of clause 13, wherein said desired hip joint angle is about 180°.
Clause 15. The orthopedic brace of clause 13, wherein said desired hip joint angle is about 90°.
Clause 16. The orthopedic brace of clause 13, 14 or 15, wherein said tensioner has a spring positioned on said intermediate portion between said cam contact segment and said second end of said tensioner, and wherein said spring has an elastic tensioning force characteristic.
Clause 17. The orthopedic brace of clause 16, wherein said spring is a first spring and said elastic tensioning force characteristic has a first value, said orthopedic brace further comprising a second spring having said elastic tensioning force characteristic with a second value different from said first value, wherein said second spring is interchangably positioned on said tensioner in place of said first spring.
Clause 18. An orthopedic brace for a body joint comprising: a first longitudinal support, a second longitudinal support and a hinge joint rotatably connecting said first and second longitudinal supports to one another, thereby rendering said first and second longitudinal supports positionable at different hinge joint angles, wherein said first longitudinal support has a first tensioner anchor and said second longitudinal support has a second tensioner anchor and a cam including a tensioner contact segment; and a tensioner attached to said first and second tensioner anchors, wherein said tensioner has a cam contact segment positioned between said first and second tensioner anchors engaging said tensioner contact segment on said cam, a spring positioned between said cam contact segment and said second tensioner anchor, and wherein said spring applies a variable elastic tensioning force to said first and second longitudinal supports as a function of a spring expansion length when said first and second longitudinal supports rotate about said hinge joint to said different hinge joint angles.
Clause 19. The orthopedic brace of clause 18, wherein said spring expansion length increases when said first and second longitudinal supports approach a hip joint angle of about 180° from a hip joint angle of about 90°.
Clause 20. The orthopedic brace of clause 18 or 19, wherein said spring expansion length decreases when said first and second longitudinal supports approach a hip joint angle of about 90° from a hip joint angle of about 180°.
Clause 21. The orthopedic brace of clause 18, 19 or 20, wherein said spring expansion length increases when said first and second longitudinal supports approach a hip joint angle of about 90°.

While the forgoing embodiments and examples consistent with the present teachings have been described and shown, it is understood that alternatives and modifications, such as those suggested and others, may be made thereto and fall within the scope. For example, it is understood that the present teachings can be applied to orthopedic braces fitting other joints of the body in addition to the hip joint by suitably modifying the hip brace in accordance with the teaching herein.

## Claims

1. An orthopedic brace for a body joint comprising:
a longitudinal support assembly having a first longitudinal support, a second longitudinal support and a hinge joint rotatably connecting said first and second longitudinal supports to one another, thereby rendering said first and second longitudinal supports positionable at different hinge joint angles, wherein said first longitudinal support has a first tensioner anchor and said second longitudinal support has a second tensioner anchor and a cam; and
a tensioner attached to said first and second tensioner anchors, wherein said tensioner follows a tensioner path extending between said first and second tensioner anchors and including a tensioner contact segment on said cam, wherein said tensioner has a cam contact segment between said first and second tensioner anchors engaging said tensioner contact segment on said cam, and wherein said tensioner applies a variable elastic tensioning force to said first and second longitudinal supports as said first and second longitudinal supports rotate about said hinge joint to said different hinge joint angles.

2. The orthopedic brace of claim 1, wherein said first longitudinal support has a first end and a second end, said second longitudinal support has a first end and a second end and said tensioner has a first end, a second end and an intermediate portion between said first and second ends of said tensioner, wherein said first end of said tensioner is attached to said first tensioner anchor, said second end of said tensioner is attached to said second tensioner anchor, wherein said hinge joint rotatably connects said second end of said first longitudinal support and said first end of said second longitudinal support, wherein said cam is at said first end of said second longitudinal support, and wherein said cam contact segment is on said intermediate portion of said tensioner.

3. The orthopedic brace of claim 2, wherein said tensioner has a spring on said intermediate portion positioned between said cam and said second tensioner anchor in said tensioner path, and wherein said spring has an elastic tensioning force characteristic.

4. The orthopedic brace of claim 3, wherein spring is a first spring and said elastic tensioning force characteristic has a first value, said orthopedic brace further comprising a second spring having said elastic tensioning force characteristic with a second value different from said first value, wherein said second spring is selectively interchangable with said first spring.

5. The orthopedic brace of claim 3 or 4, wherein said spring has a variable length varying as a function of said hinge joint angles, and wherein said variable tensioning force varies as a function of said variable length of said spring.

6. The orthopedic brace of any preceding claim, wherein said cam contact segment on said tensioner variably engages said tensioner contact segment at different positions on said cam.

7. The orthopedic brace of claim 6, wherein said variable tensioning force varies as a function of said different positions of said tensioner contact segment on said cam.

8. The orthopedic brace of any preceding claim, wherein said tensioner has a first operating orientation and said variable tensioning force substantially increases as said hinge joint angle approaches 180° from 90° at said first operating orientation of said tensioner.

9. The orthopedic brace of claim 8, wherein said variable tensioning force substantially decreases as said hinge joint angle approaches 90° from 180°at said first operating orientation of said tensioner.

10. The orthopedic brace of claim 8 or 9, wherein said tensioner is selectively transitionable from said first operating orientation to a second operating orientation and said variable tensioning force substantially increases as said hinge joint angle approaches 90° from 180° when said tensioner is in said second operating orientation.

11. The orthopedic brace of claim 10, wherein said variable tensioning force substantially decreases as said hinge joint angle approaches 180° from 90° at said second operating orientation.

12. The orthopedic brace of claim 10 or 11, wherein said second operating orientation of said tensioner is in essence a reverse of said first operating orientation of said tensioner.

13. An orthopedic brace for a body joint comprising:
a first longitudinal support, a second longitudinal support and a hinge joint, wherein said first longitudinal support has a first end, a second end and a first tensioner anchor, wherein said second longitudinal support has a first end, a second end, a second tensioner anchor and a cam at said first end of said second longitudinal support, wherein said cam has a tensioner contact segment, and wherein said hinge joint rotatably connects said first and second longitudinal supports to one another at said second end of said first longitudinal support and said first end of said second longitudinal support, thereby rendering said first and second longitudinal supports positionable at different hinge joint angles; and
a tensioner having a first end, a second end, an intermediate portion between said first and second ends and a cam contact segment on said intermediate portion, wherein said first end of said tensioner is attached to said first tensioner anchor and said second end of said tensioner is attached to said second tensioner anchor, wherein said cam contact segment engages said tensioner contact segment, wherein said tensioner applies a variable elastic tensioning force to said first and second longitudinal supports as said first and second longitudinal supports rotate about said hinge joint to said different hinge joint angles, and wherein said variable elastic tensioning force biases the longitudinal supports toward a desired position corresponding to a desired hinge joint angle.

14. The orthopedic brace of claim 13, wherein said tensioner has a spring positioned on said intermediate portion between said cam contact segment and said second end of said tensioner, and wherein said spring has an elastic tensioning force characteristic.

15. An orthopedic brace for a body joint comprising:
a first longitudinal support, a second longitudinal support and a hinge joint rotatably connecting said first and second longitudinal supports to one another, thereby rendering said first and second longitudinal supports positionable at different hinge joint angles, wherein said first longitudinal support has a first tensioner anchor and said second longitudinal support has a second tensioner anchor and a cam including a tensioner contact segment; and
a tensioner attached to said first and second tensioner anchors, wherein said tensioner has a cam contact segment positioned between said first and second tensioner anchors engaging said tensioner contact segment on said cam, a spring positioned between said cam contact segment and said second tensioner anchor, and wherein said spring applies a variable elastic tensioning force to said first and second longitudinal supports as a function of a spring expansion length when said first and second longitudinal supports rotate about said hinge joint to said different hinge joint angles.
